# EUROPEAN PATENT APPLICATION

(11) **EP 1 278 145 A1**
(43) Date of publication of application: **22.01.2003**
(21) Application number: 02015514.9
(22) Date of filing: 10.07.2002
(51) Int. Cl.: G06F 17/60

(54) **Calorie management apparatus**

(30) Priority: 16.07.2001 JP 2001215636; 16.07.2001 JP 2001215637; 16.07.2001 JP 2001215638; 16.07.2001 JP 2001215639
(71) Applicant: TANITA CORPORATION, Tokyo (JP)
(72) Inventor: Inoue, Koki, Itabashi-ku, Tokyo (JP); Ban, Teruichiro, Itabashi-ku, Tokyo (JP); Sekiguchi, Junko, Itabashi-ku, Tokyo (JP); Ishikawa, Makoto, Itabashi-ku, Tokyo (JP)
(74) Representative: Müller-Boré & Partner Patentanwälte

(57) **Abstract**

Disclosed is a calorie management apparatus for calculating and managing a calorie intake, comprising: an input unit; a storage unit; a display unit; and a control unit. According to the present invention said input unit enters the name and the amount of food ingested, and said storage unit stores the name and the calorie per unit amount of each of the foods in corresponding relation, and further stores the category to which each food belongs. The display unit displays the result of calorie intake calculated and other information about the food of which name is entered. Furthermore, said control unit controls calculation of calorie intake, based on the calorie per unit amount of the food stored in said storage unit corresponding to the name of food entered by said input unit and the amount of food also entered by said input unit.

## Description

### Background of the Invention:

### Field of the Invention:

The present invention relates to a calorie management apparatus for managing calculation of calorie intake simply by entering the name of food ingested.

### Prior Art:

Recently, it has been said in many fields that an essential factor for prevention of adult disease is to overcome the fatness of a person. Among young women a diet for beauty has become popular, and they have been interested in calorie intake and calorie consumption. There are even such persons appeared who have the body weight less than the necessity due to a one-sided diet. For the diet therapy it is necessary to calculate the calorie intake, and therefore, some portable calorie calculators have been put into market.

Among those calorie calculators there is such calculator that a personal data such as age, sex, height, body weight and life environmental condition of a user is registered in advance, and every time the user takes a meal, he enters the names of foods ingested and the food numbers each assigned to each food. Then, the calculator displays the total value of calorie intake for a day, compares it to the necessary calorie value for a day derived from the personal data, and displays the result of comparison.

Such calorie calculators generally read out the calorie data of foods stored in the storage unit for an arithmetic operation, but they necessitate entering of the data that can identify the foods ingested, as described above.

In the field of the calorie management apparatus for managing the calorie of food ingested, due to the great number of food categories present, various types of data entering method have been proposed for easily and effectively entering the intake information to the apparatus.

For example, a method for entering the food ingested has been proposed in which a code number assigned to each of the foods is entered.

Another entering method has also been known in which when the initial letter of the name of food ingested is selected from "the Japanese syllabary" then the food names starting with that letter are successively displayed for selection, as is disclosed in the Japan Patent Laid-open No. 11-211549 of the present applicant.

However, those food name entering method for prior art calorie management apparatus have several problems as follows:

In any food name entering method, irrespective of the fact that each food generally has several different names, only one such food name can be used that is stored in the apparatus, but the food names other than that can't be used for entering. For example, a food "fried chicken" is commonly called "TORI-NO-KARAAGE", but it is also called "ZANGI" in certain local area in Japan. Alternatively, someone calls it "fried chicken" using English. In such case, if the food name "TORI-NO-KARAAGE" is registered in the apparatus, entering the name "ZANGI" or "fried chicken" does not operate the apparatus to read out the stored food data for intended calorie management,

It is common practice that a person takes some particular foods for every meal. For example, a combination of rice, miso-soup and pickled vegetables is typical for every Japanese meal. In particular, the majority people take the breakfast with almost same menu. Irrespective of such fact it has been necessary to individually enter the food data into the prior art apparatus for every meal. Accordingly it was very cumbersome for the person who takes the breakfast with the same menu to enter the same food data every day. Because the person is busy in the morning in preparing for his work, the time period required for entering the food data is cumbersome for the person. If the person tries to enter the food data in latter time there may be possibility that he forgets what food and how much the food did he eat, or he simply forgets the entering operation itself. As the result the precise calorie management can't be provided. Therefore, it is preferable to enter the food data immediately after taking the meal, but it was tedious for the person to enter the food data for every meal.

On the other hand, it is possible to prepare the meal to suit for the person's taste using the food materials that the person prefers. In the prior art calorie management apparatus, however, if the person took the meal other than those generally registered in the apparatus, he had to enter the categories and the amount of the food materials used for that meal every time. Therefore, if the person took the meal made up of several food materials, it was very cumbersome to enter the data of all the materials every time.

Furthermore, the common calorie management apparatus in the prior art are designed to handle the amount of food entered in the unit of "gram" and the value of calorie in the unit of "Kcal". Accordingly, there is no problem when the precise amount in gram of each food is known, but in the case of eating away from home, for example, the user is likely at a loss what to do, because the user only knows one dish or one bowl of food he has taken, but he does not know the precise amount in gram of each food for entering into the apparatus. Of course, no precise calorie management can be performed unless the data of food ingested is entered in such circumstances.

Accordingly, in the prior art calorie management apparatus, various types of data entering method have been proposed, but they are only modification of the entering means itself, and therefore, the problems such as those described above can't be solved thereby.

Furthermore, the calorie calculator generally uses "kcal" as the unit of energy of the food. Alternatively it performs the calorie management using the pointing method with the definition of 80kcal = one point. The calorie management with such pointing method has been used especially for management of calorie intake for patients suffering from diabetes so that the allowable calorie intake for a day is limited to within the target points.

Furthermore, it has been found that not only the total calorie value, but also the balanced intake of a plurality of nutritive substance is essential for the calorie management. Therefore, the grouping of the nutritive substance and the balanced nutrition management has been done. There have been utilized various kinds of methods such as "6-Food Group" guided by the Ministry of Health and Welfare, "4-Group Pointing Method" proposed by Kagawa Nutrition University of Japan, and "Food Conversion Table" in the table 6 issued by Japanese Diabetes Academic Society.

As described above, the management of calorie intake has been conducted using "kcal" and "points" as the unit. In particular, the patient suffering from diabetes is mainly managed with the points and the physician gives him a direction using the points. However, there has frequently been seen the description using "calorie" and "kcal" in several restaurants and many teacher's manuals. Therefore, a person who conducts calorie management with the points is necessary to convert "kcal" value into the number of points or inversely to convert the remainder allowable points of calorie intake, if available, into "kcal" value. This is very cumbersome and sometimes leads to erroneous calorie management due to an error in conversion process.

Recently there is such tendency that "100kcal = 1 point" is recommended, rather than "80kcal = 1 point", for management of calorie intake with the points. This is because of facilitated conversion and easier management of calorie intake for a user, as compared to "80kcal = one point".

Accordingly, in the prior art, the management of calorie intake has been conducted by several ways, such as by the calorie unit of "kcal" and by the pointing method with "80kcal = 1 point" and "100kcal = 1 point". In particular, a person who has experienced with "80kcal = 1 point." should conduct the calorie management with "100kcal = 1 point" by additional operation of converting the conventional allowable points for calorie intake into "kcal" value and of re-converting the "kcal" value into the number of points with the conversion rate of "100kcal = 1 point". This is very cumbersome and sometimes leads to an essential problem in that any erroneous calorie management may be occurred due to an error in conversion process.

There is a plurality of grouping methods utilized for grouping the foods according to the nutritive substance. However, which of the grouping methods is used mainly depends on the user, and there may be such case happened that different grouping method is suggested every time the user has guidance. Therefore, the user is necessary to confirm the corresponding food by himself and to use the different calorie calculator suitable to each case.

In general the calorie management apparatus in the prior art has been used for managing the calorie intake, based on the allowable calorie intake for a day calculated from the personal data such as sex, age, height and body weight as well as the life style of a user, and on the allowable calorie intake directed by a physician, if any.

The remainder calorie that may be taken is calculated from the calorie intake up to now for display. Such remainder calorie, however, is not effective to the user in that he can't understand what food he can eat in how much amount.

Although the calorie management apparatus having a capability of graphically displaying the state of intake according to different groupings of the foods has been proposed, this could simply display the graph of the state of intake according to different groupings of the foods, without any assistance to the user who must select the food supplementing any lack in nutritive by himself.

There is one such apparatus put into market that displays the remainder calorie that may be taken. However, none of the prior art apparatus displays how the calorie intake or the state of intake according to different groupings of the foods is changed as the result of the certain food eaten. Therefore, there may be any possibility that the allowable calorie intake is exceeded as the result of such food eaten.

Furthermore, the calorie calculator is designed to determine or indicate whether the necessary calorie for a day is exceeded or not for the purpose of not exceeding the allowable calorie intake for a day or satisfying the requirement of calorie.

As described above, the calorie management apparatus in the prior art generally manages the calorie intake in the unit of a day. However, for diet therapy for a patient suffering from diabetes or overeating, it is essential to take a meal while paying attention to distribution of the calorie intake among the breakfast, lunch and supper.

In general it is considered desirable to evenly distribute the amount of meal among the breakfast, lunch and supper. But, in actual, it is considered ideal to distribute the calorie intake among the breakfast, lunch and supper at the ratio of 3: 4: 3. This is because if one eats too much at lunch the energy is consumed due to the activity in the daytime. In contrast thereto, any excessive calorie intake is converted into fat during sleeping so that too much supper leads to corpulence. Moreover, it has been said that a habit in which a user takes no breakfast tends to bring to such physical constitution that the fat is likely to accumulate.

In such diet therapy it is necessary to pay attention to the distribution of the calorie among three meals, in addition to the calorie intake for a day. The calorie management apparatus in the prior art provides management of calorie intake for a day, but not the distribution of calorie among the meals in a day.

Furthermore, it is considered preferable to allocate the calorie intake to the breakfast, lunch, supper and inter-meal eating at the ratio of 3: 3: 3.5: 0.5.

In view of the above an object of the present invention is to provide a calorie management apparatus having an improved data entering means for entering the data of food ingested in order to solve the prior art problem as described above.

Another object of the present invention is to provide a calorie management apparatus having an improved conversion capability for calorie intake in order to solve the prior art problem as described above.

A further object of the present invention is to provide a calorie management apparatus having a capability of confirming the state of calorie intake, which can avoid the problem in the prior art, as described above.

A yet further object of the present invention is to provide a calorie management apparatus having a capability of managing the distribution of the calorie among the meals in a day in order to solve the prior art problem as described above.

### Summary of the Invention:

According to one aspect of the present invention there is provided a calorie management apparatus for calculating and managing a calorie intake, comprising: an input unit; a storage unit; a display unit; and a control unit, wherein said input unit enters the name and the amount of food ingested, said storage unit stores the name and the calorie per unit amount of each of the foods in corresponding relation, and further stores the category to which each food belongs, said display unit displays the result of calorie intake calculated and other information about the food of which name is entered, said control unit controls calculation of calorie intake, based on the calorie per unit amount of the food stored in said storage unit corresponding to the name of food entered by said input unit and the amount of food also entered by said input unit, and if the name of food entered by the input unit is one that represents the category of the food stored in the storage unit, then said control unit causes a list of the foods belonging to that category to be displayed on the display unit so that a user selects and enters the food ingested from among the list of the foods displayed.

In one embodiment of the present invention said storage unit further stores an upper level category including a plurality of said categories, and if the name of food entered by the input unit is one that represents the upper level category stored in the storage unit, then said control unit causes a list of the categories belonging to that upper level category to be displayed on the display unit.

According to another aspect of the present invention there is provided a calorie management apparatus for calculating and managing a calorie intake, comprising: an input unit; a food group name registration unit; a storage unit; a control unit; and a display unit; wherein said input unit enters the name and the amount of food ingested; said food group name registration unit enters a new desired set name for a food group including a plurality of foods, said storage unit stores the name and the calorie per unit amount of each of the foods in corresponding relation, and further stores the set name for the food group including a plurality of foods entered by said food group name registration unit and the corresponding calorie value, said control unit controls calculation of calorie intake, based on the calorie per unit amount of the food stored in said storage unit corresponding to the name of food entered by said input unit and the amount of food also entered by said input unit, and allows entering of the food ingested with the new set name for the food group by said input unit, and said display unit displays the result of calorie intake calculated and other information about the food of which name is entered.

In one embodiment of the present invention said storage unit further stores a basic unit volume as the unit representing the amount of the food, and said control unit further controls calculation of calorie of the food ingested, based on the basic unit volume of the food if this is entered by the input unit.

In another embodiment of the present invention said display unit displays the basic unit volume, weight and calorie of each food, and when either one of the basic unit volume, weight or calorie of the food is entered by the input unit then the control unit causes calculation of the other values than that entered if necessary for displaying them on the display unit.

According to further aspect of the present invention there is provided a calorie management apparatus for calculating and managing a calorie intake, comprising: an input unit; a storage unit; a control unit; and a display unit, wherein said input unit enters the name and the amount of food ingested; said storage unit stores the name and the calorie per unit amount of each of the foods in corresponding relation, said control unit controls calculation of calorie intake, based on the calorie per unit amount of the food stored in said storage unit corresponding to the name of food entered by said input unit and the amount of food also entered by said input unit, while the name and the amount of the food stored in said storage unit is displayed, said control unit causes confirmation by calculating the calorie intake that would be resulted if such food is eaten, and said display unit displays the result of calorie intake calculated and other information about the food of which name is entered.

In one embodiment of the present invention the calorie management apparatus further includes a graphic display unit that graphically illustrates the state of calorie intake for each of food groups after taking a meal.

According to yet further aspect of the present invention there is provided a calorie management apparatus for calculating and managing a calorie intake, comprising: an input unit; a storage unit; a control unit; and a display unit, wherein said input unit enters the name and the amount of food ingested; said storage unit stores the name and the calorie per unit amount of each of the foods in corresponding relation, and further stores the information about contents of nutritive substance classified by food grouping for each of the foods, said display unit displays the result of calorie intake calculated and other information about the food of which name is entered, and said control unit controls calculation of calorie intake, based on the calorie per unit amount of the food stored in said storage unit corresponding to the name of food entered by said input unit and the amount of food also entered by said input unit, for calorie intake management according to a plurality of food groups.

In one embodiment of the present invention the calorie management apparatus further includes a graphic display unit that graphically illustrates the state of calorie intake for each of food groups.

### Brief Description of the Drawings:

The present invention will be described in more detail with reference to the accompanying drawings, in which:
Fig. 1 is a perspective view of a calorie management apparatus according to one embodiment of the present invention;
Fig. 2 is a view illustrating the calorie management apparatus in Fig. 1, but at the condition when the cover is closed and the touch pen is stored;
Fig. 3 is a block diagram illustrating a circuit configuration of the calorie management apparatus in Fig. 1;
Fig. 4 is a flow chart of a main routine for the calorie management apparatus in Fig. 1;
Fig. 5 is a flow chart illustrating an initial setting routine for the calorie management apparatus in Fig. 1;
Fig. 6 is a flow chart illustrating a meal input mode for the calorie management apparatus in Fig. 1;
Fig. 7 is a flow chart illustrating the Japanese syllabary-retrieving mode for the calorie management apparatus in Fig. 1;
Fig. 8 is a flow chart illustrating a food data display mode for the calorie management apparatus in Fig. 1;
Fig. 9 is a flow chart illustrating a basic unit volume modification mode for the calorie management apparatus in Fig. 1;
Fig. 10 is a flow chart illustrating a confirmation mode for the calorie management apparatus in Fig. 1;
Fig. 11 is a flow chart illustrating a set name-retrieving mode for the calorie management apparatus in Fig. 1;
Fig. 12 is a flow chart illustrating a set name-forming mode for the calorie management apparatus in Fig. 1;
Fig. 13 is a flow chart illustrating a balance mode for the calorie management apparatus in Fig. 1;
Fig. 14 is a flow chart illustrating a converted display mode for different type food classification for the calorie management apparatus in Fig. 1;
Fig. 15 is a flow chart illustrating a food supplement mode for the calorie management apparatus in Fig. 1;
Fig. 16 is a flow chart illustrating a three-meal balance mode for the calorie management apparatus in Fig. 1;
Fig. 17 is a view illustrating a normal screen on a touch panel display of the calorie management apparatus in Fig. 1;
Fig. 18 is a view illustrating a display screen during the initial setting routine for the calorie management apparatus in Fig. 1;
Fig. 19 is a view illustrating a display screen during the life style setting mode for the calorie management apparatus in Fig. 1;
Fig. 20 is a view illustrating a display screen during the calorie value confirmation mode for the calorie management apparatus in Fig. 1;
Fig. 21 is a view illustrating a display screen during the unit setting mode for the calorie management apparatus in Fig. 1;
Fig. 22 is a view illustrating a display screen during the meal input mode for the calorie management apparatus in Fig. 1;
Fig. 23 is a view illustrating a display screen during the Japanese syllabary retrieving mode for the calorie management apparatus in Fig. 1;
Fig. 24 is a view illustrating a display screen during the voice input mode for the calorie management apparatus in Fig. 1;
Fig. 25 is a view illustrating a display screen when the voice is recognized during the voice input mode for the calorie management apparatus in Fig. 1;
Fig. 26 is a view illustrating a display screen during the food data display mode for the calorie management apparatus in Fig. 1;
Fig. 27 is a view illustrating a display screen when the basic unit volume key is depressed in the calorie management apparatus in Fig. 1;
Fig. 28 is a view illustrating a display screen on which the result of arithmetic operation in the confirmation mode is displayed in the calorie management apparatus in Fig. 1;
Fig. 29 is a view illustrating a display screen during the set menu meal name-retrieving mode for the calorie management apparatus in Fig. 1;
Fig. 30 is a view illustrating a display screen on which contents of the set menu meal are displayed in the calorie management apparatus in Fig. 1;
Fig. 31 is a view illustrating a display screen during set menu meal name-forming mode for the calorie management apparatus in Fig. 1;
Fig. 32 is a view illustrating a display screen during the balance mode for the calorie management apparatus in Fig. 1;
Fig. 33 is a view illustrating a display screen for selecting the type of food classification used for converted display for the calorie management apparatus in Fig. 1;
Fig. 34 is a view illustrating a display screen during the food supplement mode for the calorie management apparatus in Fig. 1;
Fig. 35 is a view illustrating a display screen on which a list of foods preferable for supplement of lack in nutrition is displayed in the calorie management apparatus in Fig. 1;
Fig. 36 is a view illustrating a display screen during the three-meal balance mode for the calorie management apparatus in Fig. 1;
Fig. 37 is a view illustrating a grouping method of 5-group type in which the foods are classified into five groups according to nutritive substance;
Fig. 38 is a view illustrating another grouping method known as 4-group pointing method; and
Fig. 39 is a view illustrating a further grouping method in which the foods are classified into six groups according to nutritive substance.

### Description of the Preferred Embodiment:

Before proceeding to the detailed description of an embodiment of the present invention, the fundamental features of a calorie management apparatus of the present invention will be summarized below:
Firstly, the calorie management apparatus of the present invention has a capability of entering the names of foods, not only by the limited food names registered in the apparatus in advance, but also by the categories (or genres) of the foods;
Secondly, instead of entering, every time, all the food data of the foods frequently ingested, they can easily be entered to omit the laborious operation for one's meal; and
Thirdly, the amount of food ingested may be entered in any unit understandable for a user.

The term "category (or genre)" as used herein is defined as one that is classified by an attribute of the food. For example, a cookie, a chocolate, a doughnut, etc., belong to the category of "confectionary", and a chicken curry, a chicken-burger, a fried chicken, etc., belong to the category of "chicken food".

The term "basic unit volume" as used herein is defined as one that represents a certain kind of unit amount of food such as "one piece", "one sheet", "one dish", "one bowl", "one slice", etc.

Fourthly, the calorie management apparatus of the present invention makes possible to manage the calorie intake with both conversion rate of "80kcal = 1 point" and "100kcal = 1 point".

Fifthly, the calorie management apparatus of the present invention makes possible to manage the calorie intake according to a plurality of food grouping methods based on the nutritive substance.

Sixthly, the calorie management apparatus of the present invention makes possible to indicate what food a user can eat in how much amount, based on the remainder calorie that may be taken.

Seventhly, the calorie management apparatus of the present invention makes possible for a user to know the remainder calorie that he may further take, the total calorie intake resulted if the user would eat the desired food, and the state of intake according to different groupings of the foods.

Finally the calorie management apparatus of the present invention makes possible to manage the distribution of the calorie among the meals in a day, which allows balanced calorie intake for each of the meals.

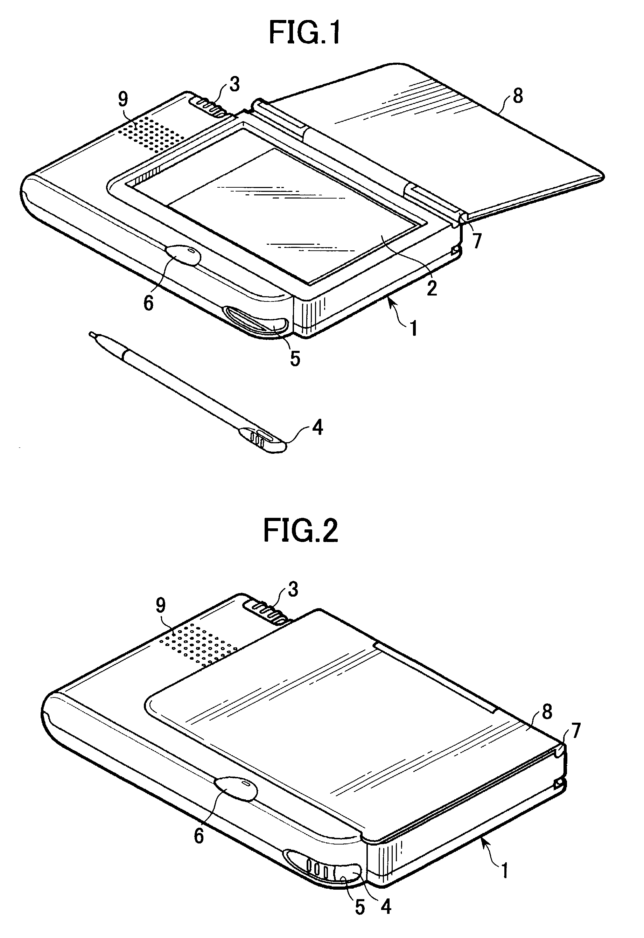

Fig. 1 is a perspective view of a calorie management apparatus according to one embodiment of the present invention. Referring to Fig. 1, the calorie management apparatus 1 comprises a touch panel 2 mounted on an upper surface of the apparatus for entering and displaying the data, and a power switch 3 formed by a slide switch that is slid for turning the apparatus ON. The apparatus further comprises a touch pen 4 that is held within a touch pen container 5 when the apparatus is not used. The touch panel 2 displays the food menus and the result of arithmetic operation and selects any one of the displayed food menus by touching it with the touch pen 4. At the lower portion of the upper surface of the apparatus 1 a voice input aperture 6 is formed and a microphone is mounted therein. A cover 8 is provided on the apparatus via a hinge 7 for freely moving to an open or closed position, and the touch panel 2 is covered with the cover 8 when it is at closed position. An array of several projections 9 is provided on the apparatus 1 at the location where a thumb finger of a left hand is positioned when the apparatus 1 is held by the left hand in order to prevent the apparatus 1 from slipping off. In this way the calorie management apparatus 1 has a size suitable for a user to carry with him for use outside of his home.

Fig. 2 is a view illustrating the calorie management apparatus 1 in Fig. 1, but at the condition when the cover 8 is closed. In such condition the touch pen 4 has been received in the touch pen container 5.

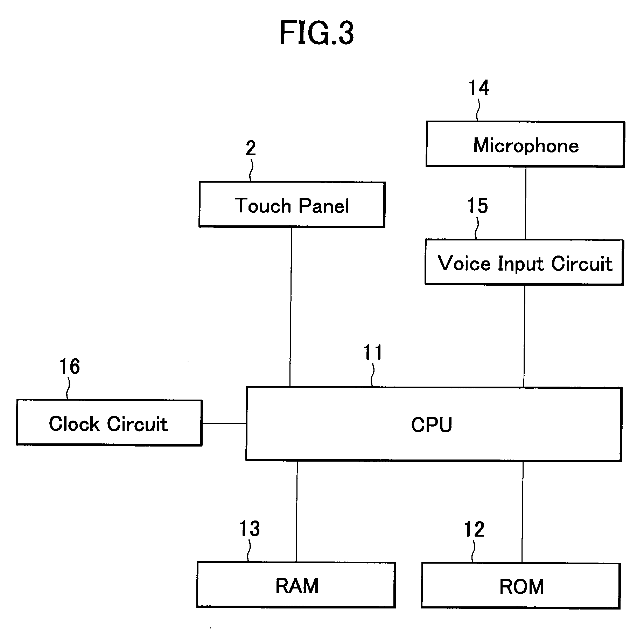

Fig. 3 is a block diagram illustrating a circuit configuration of the calorie management apparatus 1. Referring to Fig. 3, the calorie management apparatus 1 comprises a touch panel 2, a CPU 11 for processing on the basis of a program, a ROM 12 for storing various data necessary for the calorie management, such as an arithmetic program, the names of foods and the corresponding values of calorie per unit amount of foods, and voice signals for food names, etc., a RAM 13 for temporally storing the personal body information entered and the data being processed, a microphone 14 for voice input, a voice input circuit 15 for converting the voice signal from the microphone 14 from an analog format to a digital format, and a clock circuit 16 for managing the date and time. The CPU 11 has a voice recognition capability for determining whether there is any food name stored in the apparatus that corresponds to or is similar to the digital voice signal.

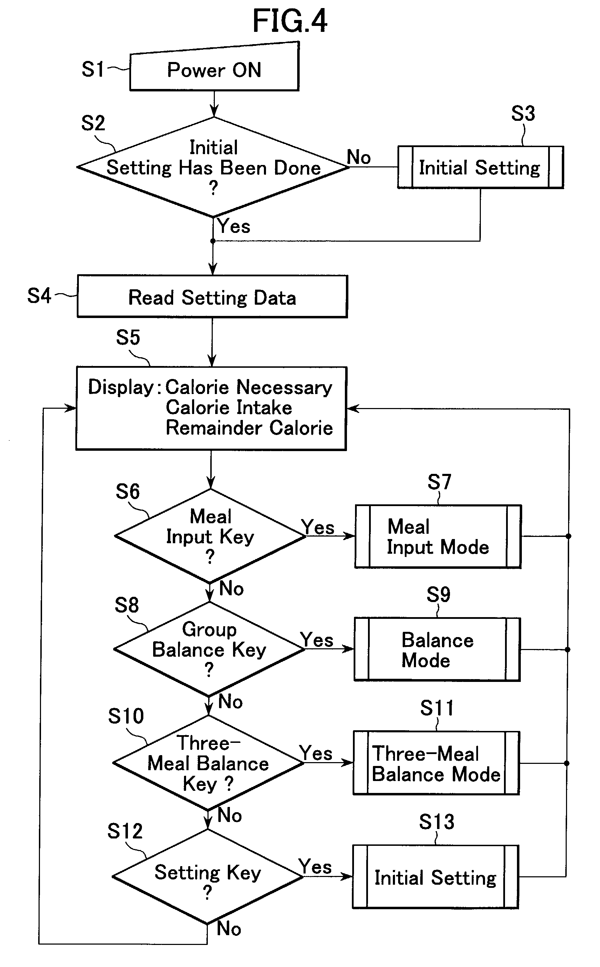

Now, the detailed description of how to use and how to operate the calorie management apparatus having the configuration as above will be made in conjunction with the flow charts of Figs. 4 to 16, and Figs. 17 to 36 illustrating an LCD display screen. The term "key" as used herein means such key that is displayed on the touch panel LCD module 2 and is operated by touching it with the touch pen 4.

Fig. 4 is a flow chart of a main routine for the calorie management apparatus 1 of the present invention. It is assumed that the cover 8 is opened and the touch pen 4 is taken out of the container 5.

The power switch 3 is slid to turn ON the calorie management apparatus 1 (Step S1) Then, the CPU 11 checks to determine whether the personal data of a user is stored in the RAM 13 (Step S2), and if not, the routine proceeds to an initial setting step (Step S3). But, if so, the CPU 11 reads the personal data (Step S4).

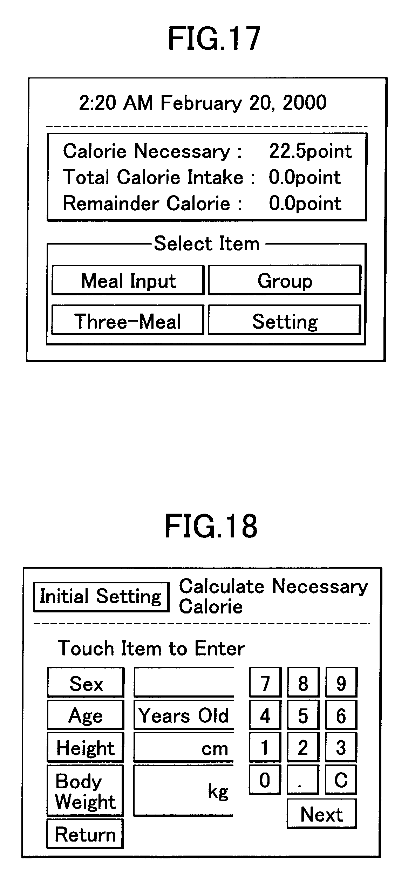

Fig. 17 is a view of a normal display screen. It displays a calorie intake necessary for a day for a user, a calorie intake up to the current time in that day, and the remainder calorie allowed to take for that day (Step S5). In the lower portion of the display screen "a meal input key", "a group balance key", "a three-meal balance key" and "a setting key" are displayed, while in the upper portion thereof the current time and date is displayed.

When the meal input key is depressed (Step S6) the routine proceeds to a meal input mode (Step S7). When the group key is depressed (Step S8) the routine proceeds to a balance mode where the calorie intake is displayed according to the groups of foods (Step S9). When the three-meal key is depressed (Step S10) the routine proceeds to three-meal balance mode where the calorie intake of three meals, i.e., breakfast, lunch and supper, and that of an inter-meal eating are displayed (Step S11).

When the setting key is depressed (Step S12), the routine proceeds to the initial setting mode (Step S13). If none of the keys is depressed the routine returns to Step S5 where the display screen as shown in Fig. 17 continues to be displayed.

While not shown in the flow chart, sliding the power switch 3 in any state or in any mode causes the calorie management apparatus 1 to be turned OFF. While also not shown in the flow chart, the clock circuit 16 executes another routine for clocking the current time for time and date management, independent of the main routine.

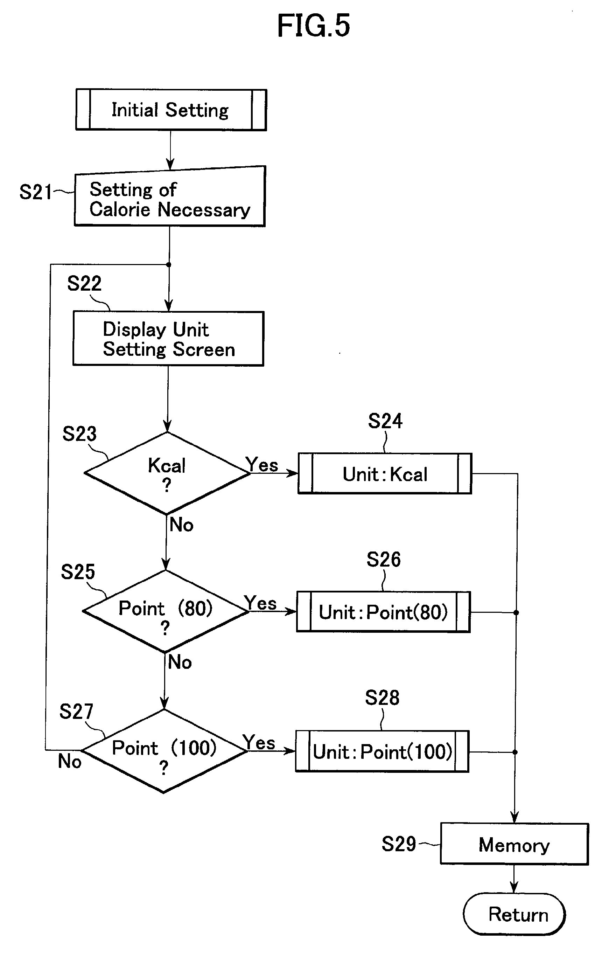

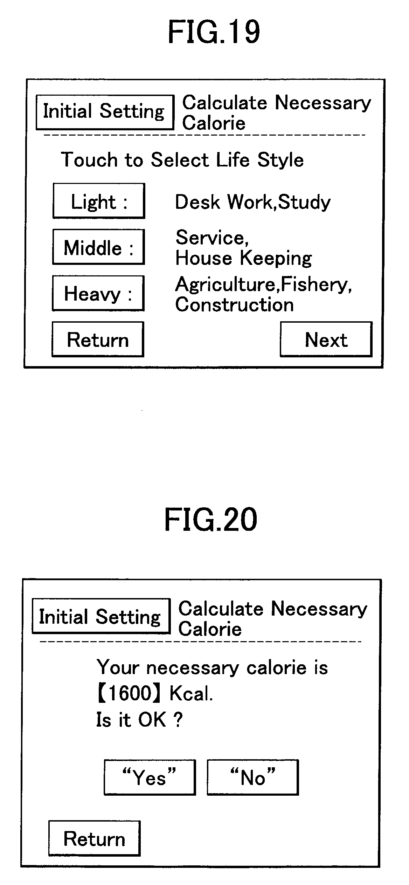

The initial setting routine will be described in detail. Fig. 5 is a flow chart illustrating the initial setting routine. Fig. 18 is a view illustrating a display screen during the initial setting routine. A user successively enters the data of sex, age, height and body weight by touching a ten-key pad displayed on the right-hand side of the screen. Then the user depresses a "Next" key to display a life style selection screen, as shown in Fig. 19. Depending on the data entered to this screen the CPU 11 calculates the calorie value necessary for a day for the user and prompts to confirm the calorie value, as shown in Fig. 20. If the user depresses a "No" key the display returns to the screen, as shown in Fig. 18, and then, the user performs the setting once again (Step S21). The method of calculating calorie value necessary for a day can be seen in many literatures, and therefore, the detailed description thereof is omitted here.

The calorie value necessary for a day that is calculated is then allocated to each of the meals. According to the present invention the calorie value is allocated to breakfast, lunch, supper, and some inter-meal eating at the ratio of 3 : 3 : 3.5 : 0.5. For example, assuming that the calorie intake necessary for a day is 2000Kcal, it is allocated in 600 : 600 : 700 : 100 (Kcal). Those calorie values necessary for each of the meals are used in the three-meal balance mode described hereafter.

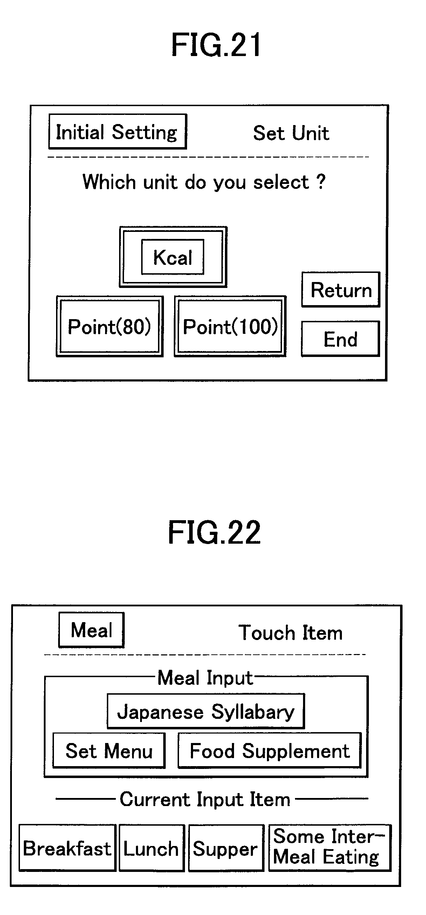

Next, a unit setting screen is displayed, as shown in Fig. 21 (Step S22). Here, any one of "Kcal", "Point (80)" or "Point (100)" key is selected for use as the unit of calorie value. If "Kcal" key is selected it is directly used. However, if "Point (80)" or "Point (100)" key is selected, 80Kcal or 100Kcal is taken as one point according to "Calorie Point Method", and the calorie management is performed based on the number of points.

Therefore, if "Kcal" key is depressed (Step S23), the Kcal is used as the unit of the calorie value (Step S24). If "Point (80)" key is depressed (Step S25) the point 80 is used as the unit of the calorie value (Step S26). If "Point (100)" key is depressed (Step S27) the point 100 is used as the unit of the calorie value (Step S28). The setting information is stored in RAM 13 (Step S29). Then the subsequent display of the calorie value is performed based on this setting information.

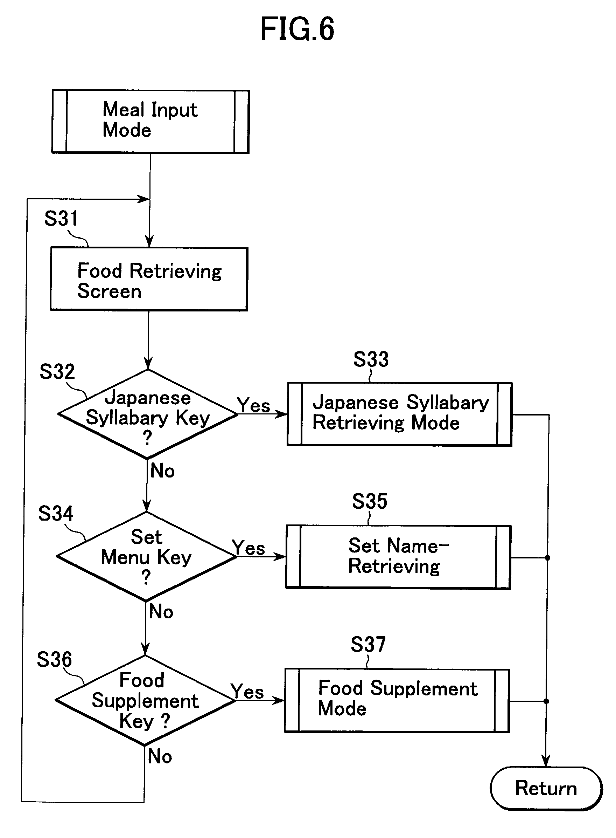

Next, the meal input mode will be described in detail. Fig. 6 is a flow chart illustrating the meal input mode. The food input method selection screen is displayed (Step S31). Fig. 22 is a view illustrating a display screen during the meal input mode. If the Japanese syllabary key is depressed (Step S32) the routine proceeds to the Japanese syllabary retrieving mode (Step S33). If the set menu key is depressed (Step S34) the routine proceeds to the set name-retrieving mode for retrieving the name of set menu (or fixed menu) meal (Step S35). If the food supplement key is depressed (Step S36) the routine proceeds to the food supplement mode (Step S37). If none of the keys is depressed the input method selection screen continues to be displayed. Although not shown in the flow chart, depressing the "current input item" displayed on the lower portion of the screen in Fig. 22 causes selection of any one of breakfast, lunch, supper and some inter-meal eating for calorie intake management.

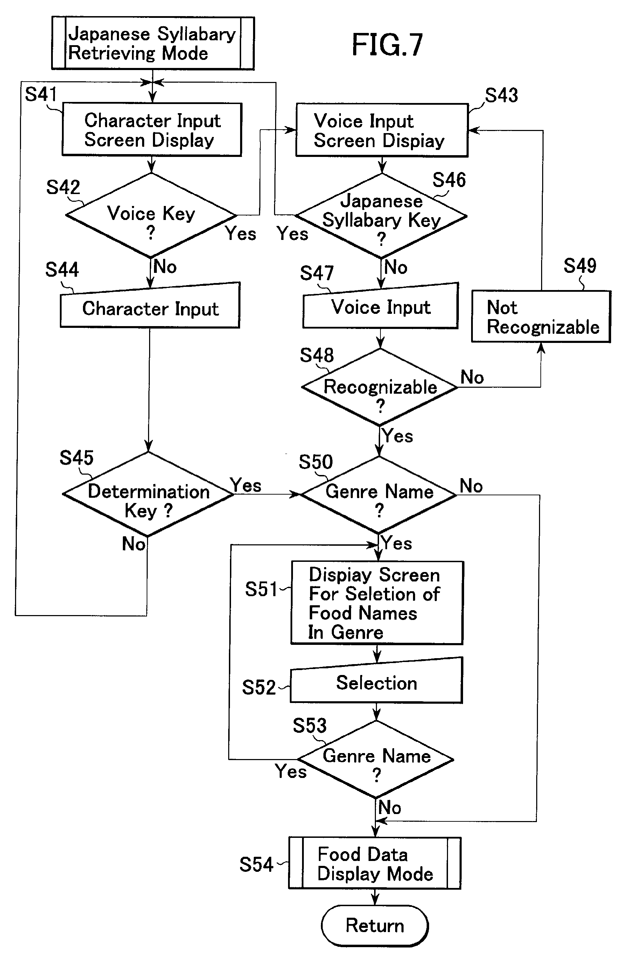

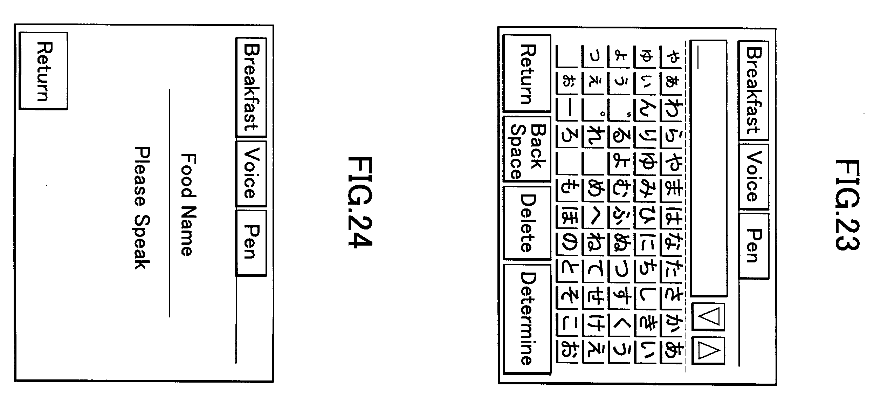

Now, the Japanese syllabary retrieving mode will be described in more detail. Fig. 7 is a flow chart illustrating the Japanese syllabary retrieving mode. The Japanese syllabary input screen is displayed (Step S41). Fig. 23 is a view illustrating a display screen during the Japanese syllabary retrieving mode. A check is made to determine whether the voice key is depressed (Step S42). If not, the character input is accepted, but if so, the routine proceeds to the voice input mode (Step S43).

In the pen touch input mode the Japanese syllabary from 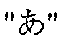 to 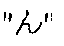 is displayed, as shown in Fig. 23. In such condition the user inputs the food name one character by one character using the touch pen 4 (Step S44). Each time one character is entered a check is made to determine whether the food name has been entered, based on the depression of a determination key (Step S45). A "back space" key, if depressed, erases one character entered on the screen, and a "delete" key, if depressed, erases all the characters entered on the screen. The Japanese syllabary input screen continues to be displayed until the input operation is ended.

Next, the voice input will be summarized before the detailed description of the voice input mode. In the voice input mode the voice recognition process is used to select the name of food ingested from among the food names registered in the ROM 12. Any commonly known voice recognition technology may be used that has already been utilized in various fields such as a telephone, a personal computer and a car navigation system. Therefore, the voice recognition technology itself is not further described here, but it is suffice to say as follows:

The voice recognition is performed in the flowing process, for example. At first, an incoming voice is received and converted into an electrical signal in a microphone. In this case, in order not to take up any surrounding noise, the close-talking microphone or small directional microphone is preferably used for the microphone. However, in view of such capability of CPU 11 that any noise can be removed via a software process, there is no need, here, to limit the type of microphone to any specified one. The voice signal entered through the microphone is then fed to a frequency analysis section where it is divided into frames each having time duration of a few ms to a few tens ms and the spectrum for each frame is calculated. The spectral analysis is performed with Fast Fourier Transform. The resulting spectrum is converted into parameters based on the auditory measure and is subjected to noise remove process. The changing pattern of the spectrum with the time is compared in a phoneme recognition section to a phoneme model expressing the time series of voice parameters, and then, the result of the phoneme recognition is compared in a word recognition section to a word model for calculating the degree of matching therebetween. The result of word recognition is used to select a series of words matching the word model.

In the voice input mode executed in Step S43 a display screen as shown in Fig. 24 is displayed. If the pen input key is depressed the routine proceeds to the pen input mode in Step S41 (Step S46). In voice input mode the LCD 2 displays the screen as shown in Fig. 24. The user who wants to manage his calorie intake speaks the name of food ingested so that the voice reaches the microphone 14 in the voice input aperture 6 (Step S47). It is assumed, here, that the user speaks "PAN (a bread)".

The voice signal entered through the microphone 14 is converted from the analog signal to the digital signal in the voice input circuit 15. Then the voice recognition section 11 recognizes which of the voice signals representing the food names stored in the ROM 12 is similar to that digital voice signal (Step S48). If such recognition is successfully done the names of foods are sequentially displayed on the LCD 2 in the order of highest degree of matching to the voice signal "PAN (a bread)" (Step S51).

On the other hand, if the user could not find the food name that he spoke in the display on the LCD 2, it is determined that the voice recognition is failed in Step S48, and such fact is displayed on the LCD 2 (Step S49). Then the routine returns to the voice input mode in Step S43.

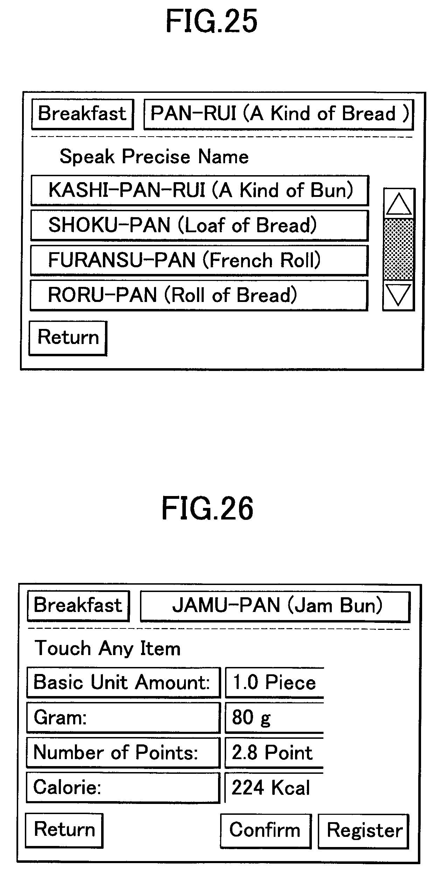

If the character input is ended in Step S45 or the voice signal is recognizable in Step S48 then the routine proceed to the Step S50 where a check is made to determine whether the food name is the category name (or genre name). If so, a list of the foods belonging to that category is displayed, as shown in Fig. 25 (Step S51). Then the user may select the food that he desires among the list with the touch pen 4 (Step S52). If the food name thus selected is also the category name then the routine proceeds to Step S51 where another list of the foods belonging to that category is displayed (Step S53). In this example, a first input of food name "PAN (a bread)" is made and a list of foods belonging to "PAN (bread)" is displayed. If "KASHI-PAN (a bun)" is selected among the list then another list of the foods belonging thereto is displayed.

If the food name entered is not category name in Step S50 or the food name selected is not category name in Step S53 then the routine proceeds to the food data display mode where the data of the food is displayed (Step S54).

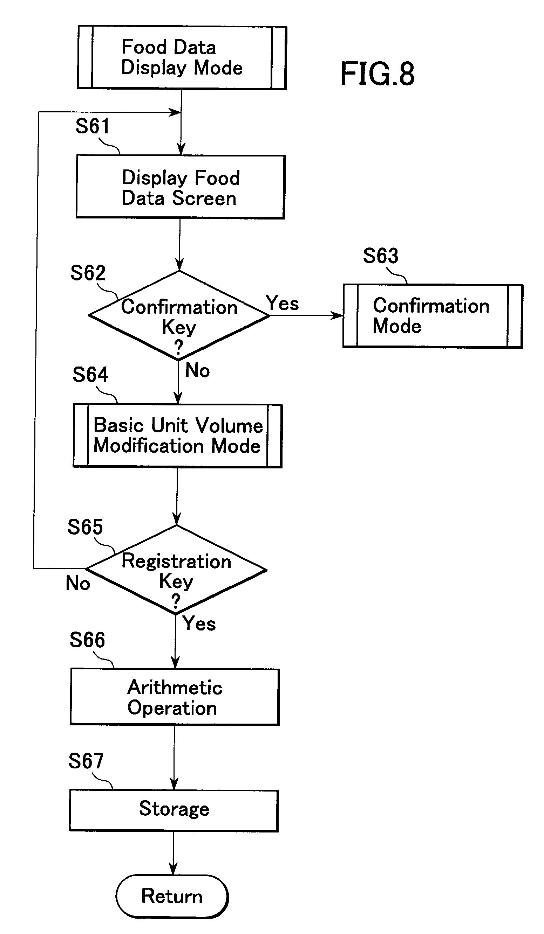

Next, the food data display mode will be described in detail. Fig. 8 is a flow chart illustrating the food data display mode. A display screen as shown in Fig. 26 is produced (Step S61). Then, a check is made to determine whether a confirmation key is depressed or not (Step S62). If so, the routine proceeds to a confirmation mode (Step S63), but if not, the routine proceeds to a basic unit volume modification mode (Step S64). Then, a check is made to determine whether a registration key is depressed or not (Step S65). If so, an arithmetic operation for calculating the calorie intake for that day is executed, assuming that the food currently displayed has been ingested in that amount (Step S66). Then, the result of calculation is stored in the RAM 13 (Step S67).

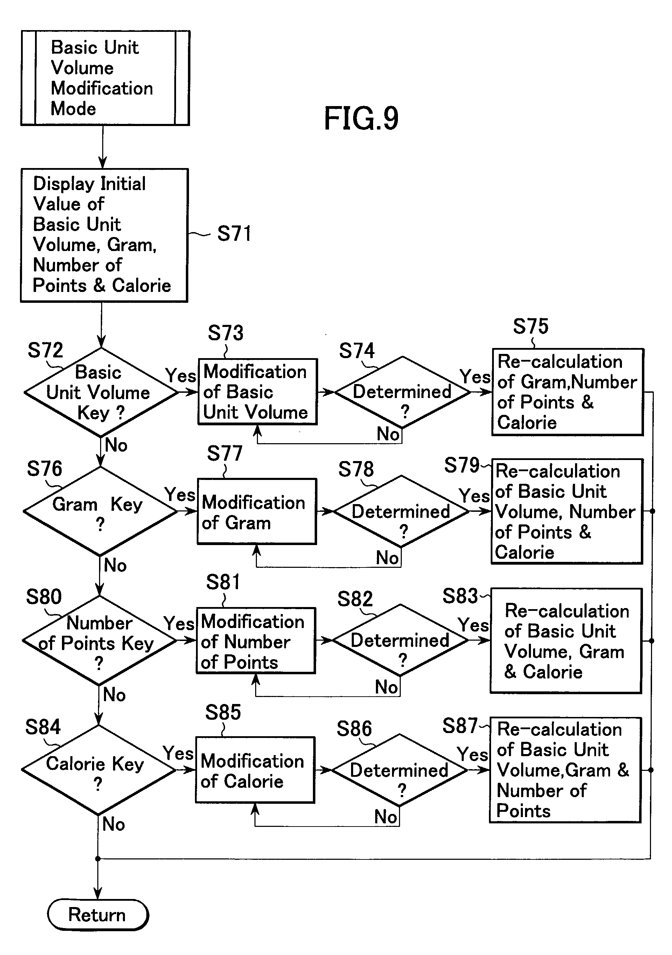

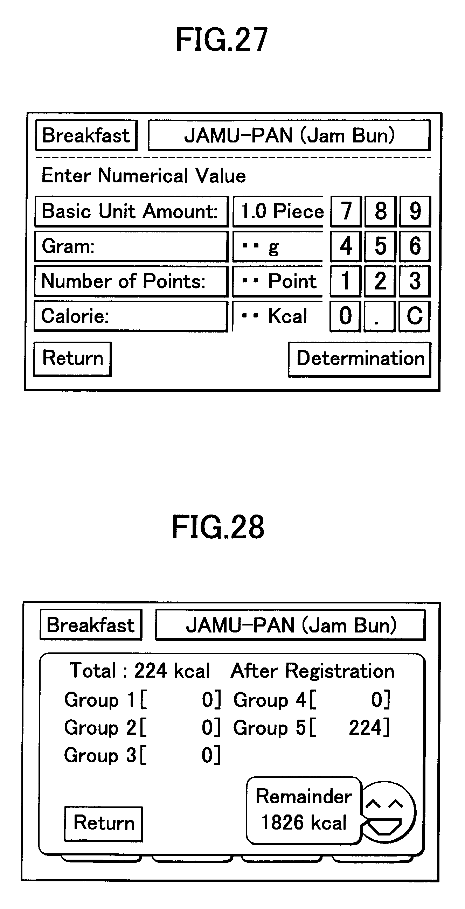

Next, the basic unit volume modification mode will be described in more detail. Fig. 9 is a flow chart illustrating the basic unit volume modification mode. A display screen as shown in Fig. 26 is produced (Step S71). If a basic unit volume key is depressed (Step S72), numeral keys are displayed for modifying the value of the basic unit volume (Step S73), as shown in Fig. 27. When the user enters the numeral value and depresses the determination key (Step S74) an arithmetic operation for calculating the gram value, the number of points and the calorie value for that basic unit volume is executed (Step S75).

If a gram key is depressed (Step S76) then the modification of the gram value is executed in the same manner (Step S77). In particular, when the user enters the gram value and depresses the determination key (Step S78) an arithmetic operation for calculating the basic unit volume, the number of points and the calorie value for that gram value is executed (Step S79). If a number of points key is depressed (Step S80) then the modification of the number of points is executed in the same manner (Step S81). In particular, when the user enters the value of number of points and depresses the determination key (Step S82) an arithmetic operation for calculating the basic unit volume, the gram value and the calorie value for that number of points is executed (Step S83). If a calorie key is depressed (Step S84) then the modification of the calorie value is executed in the same manner (Step S85). In particular, when the user enters the numerical value and depresses the determination key (Step S86) an arithmetic operation for calculating the basic unit volume, the number of points and the gram value for that calorie value is executed (Step S87).

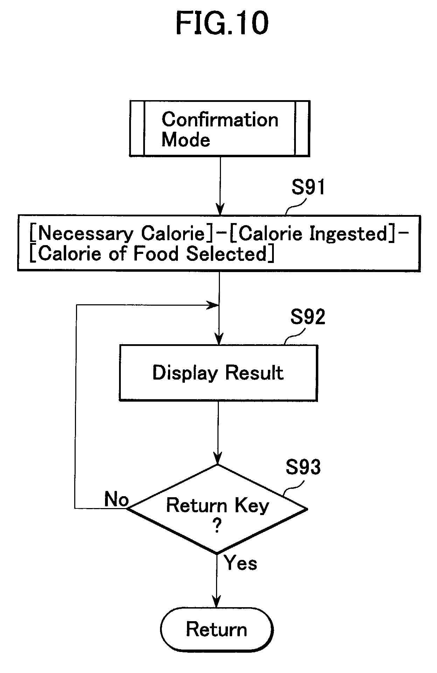

Next, the confirmation mode will be described in more detail. Fig. 10 is a flow chart illustrating the confirmation mode. In this confirmation mode, an arithmetic operation is performed for calculating the total calorie intake after taking the amount of food selected based on the remainder calorie allowed to take for that day (Step S91). The result of calculation is displayed, as shown in Fig. 28 (Step S92). If the return key is depressed the confirmation mode is ended (Step S93).

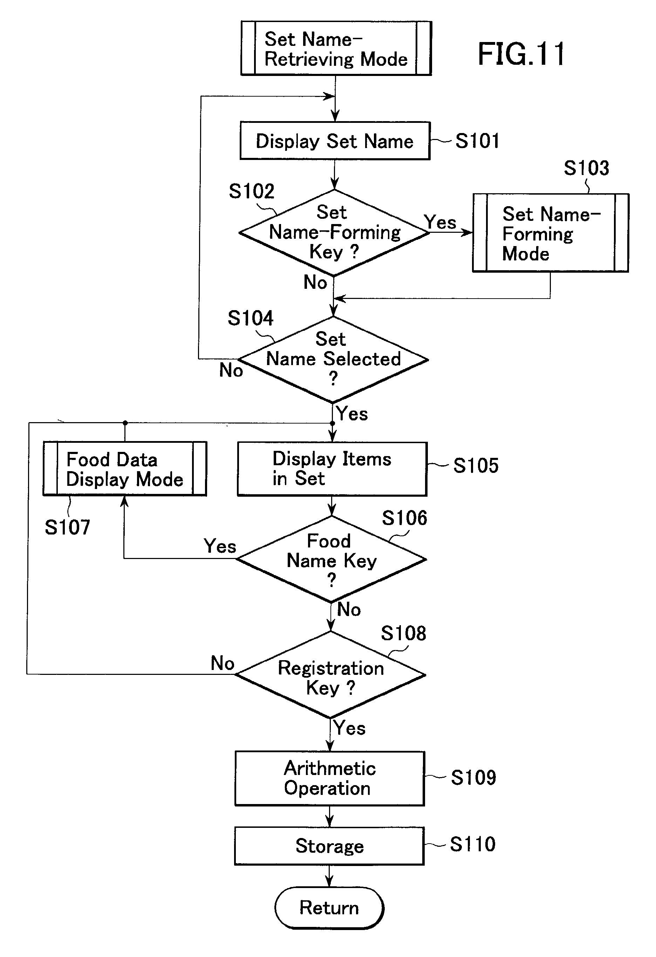

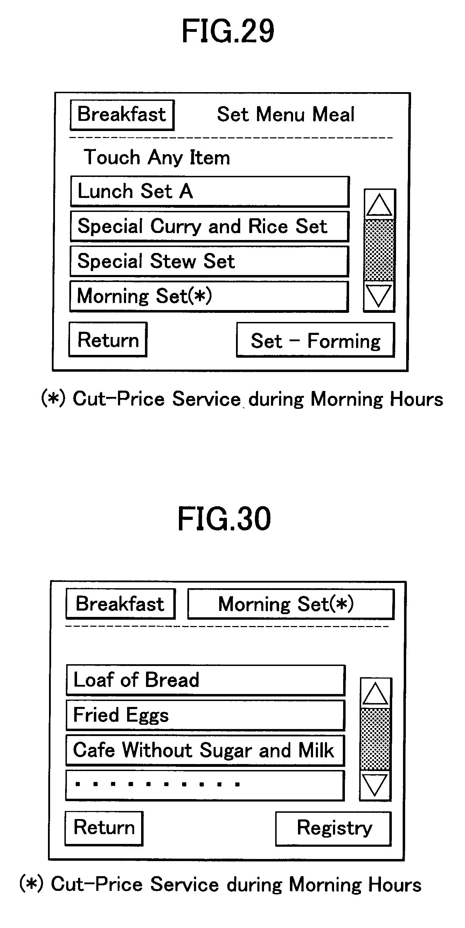

Next, the set name-retrieving mode for retrieving the name of set menu (or fixed menu) meal will be described in more detail. Fig. 11 is a flow chart illustrating the set name-retrieving mode. The display screen for displaying a list of set names (or names of set or fixed menu meals) is produced, as shown in Fig. 29 (Step S101). A check is made to determine whether a set name-forming key is depressed or not (Step S102). If so, the routine proceeds to a set name-forming mode (Step S103), but if not, a check is made to determine whether any set name is selected among the list of set names (Step S104). If not, the routine returns to Step S101 for displaying the list of set names.

If certain set name is selected then items or foods included in that set menu meal are displayed, as shown in Fig. 30 (Step S105). If any item is selected therefrom (Step S106) the routine proceeds to a food data display mode (Step S107), but if no item is selected the routine proceeds to Step S108 where a check is made to determine whether the registration key is depressed or not. If not, the routine returns to Step S105 where the items or foods included in that set menu meal are displayed again. But if yes in Step S108, indicating that the set menu meal is taken, then the routine proceeds to Step S109 where an arithmetic operation for adding the calorie value for the items included in that set menu meal to produce the total calorie intake and nutritive substance for that day, which is then stored in the RAM 13 (Step S110).

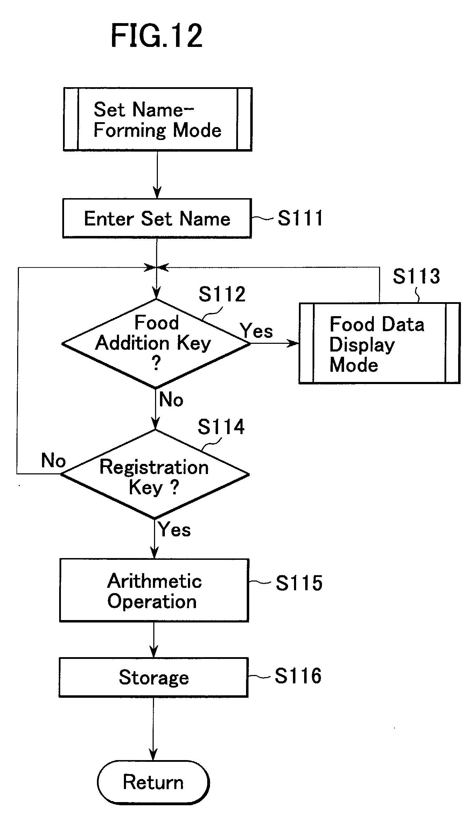

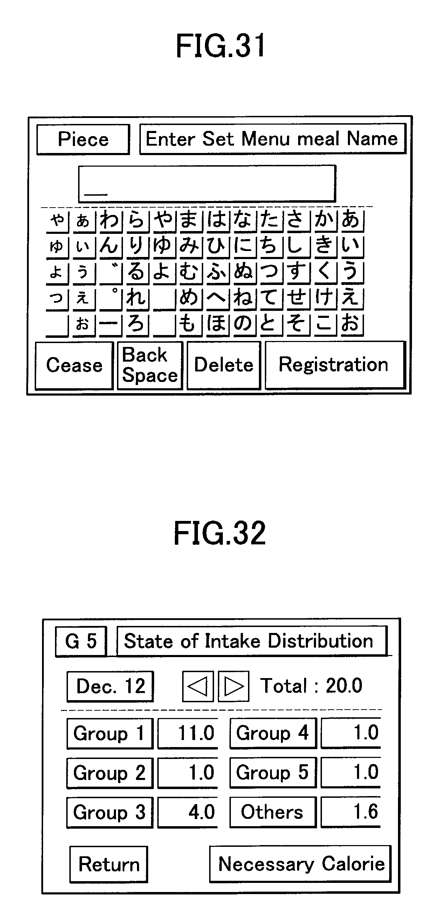

Next, the set name-forming mode will be described in more detail. Fig. 12 is a flow chart illustrating the set name-forming mode. A message for prompting the user to enter the set name (or the name of set or fixed menu meal) is displayed, as shown in Fig. 31 (Step Sill). When the set name is entered then a list of the foods added as that set menu meal is displayed. Then a check is made to determine whether a food addition key is depressed or not (Step S112). If yes, the routine proceeds to the food data display mode (Step S113). However, if the registration key is depressed (Step S114), then an arithmetic operation for calculating the calorie for each of foods added and total calorie for that set menu meal as well as the nutritive substance is performed (Step S115), and the result is stored in the RAM 13 (Step S116).

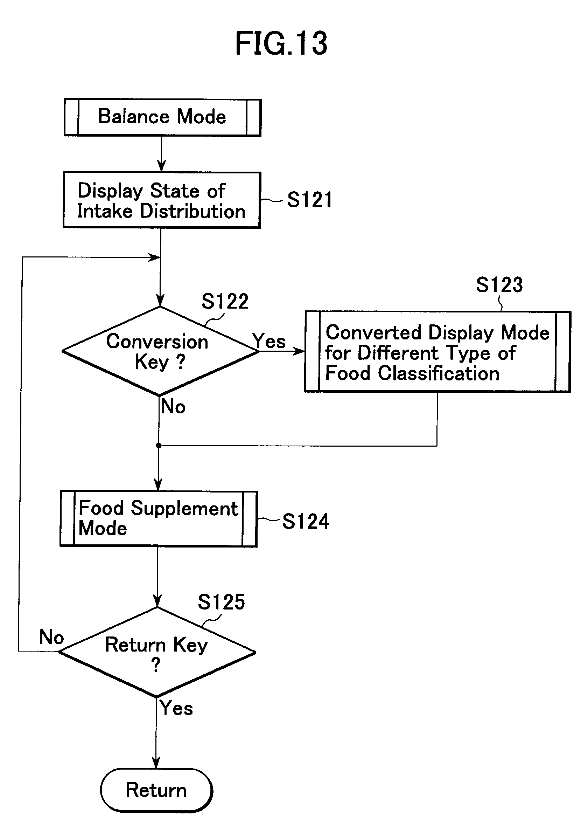

Next, the balance mode will be described in more detail. Fig. 13 is a flow chart illustrating the balance mode. Fig. 32 is a screen view illustrating the state of intake distribution in the form of "G5" or 5-group type of food classification depending on the nutritive substance to show the distribution of foods among those groups for the day (Step S121). If a conversion key is depressed (Step S122) then the routine proceeds to any converted display mode for different type of food classification (Step S123). But, if not, the routine proceeds to the food supplement mode (Step S124). Thereafter, the state of intake distribution is kept displayed in the currently used food classification format until a return key is depressed (Step S125). The term "others" in Fig. 32 refers to such food that does not belong to any of the 5 groups, as described hereafter.

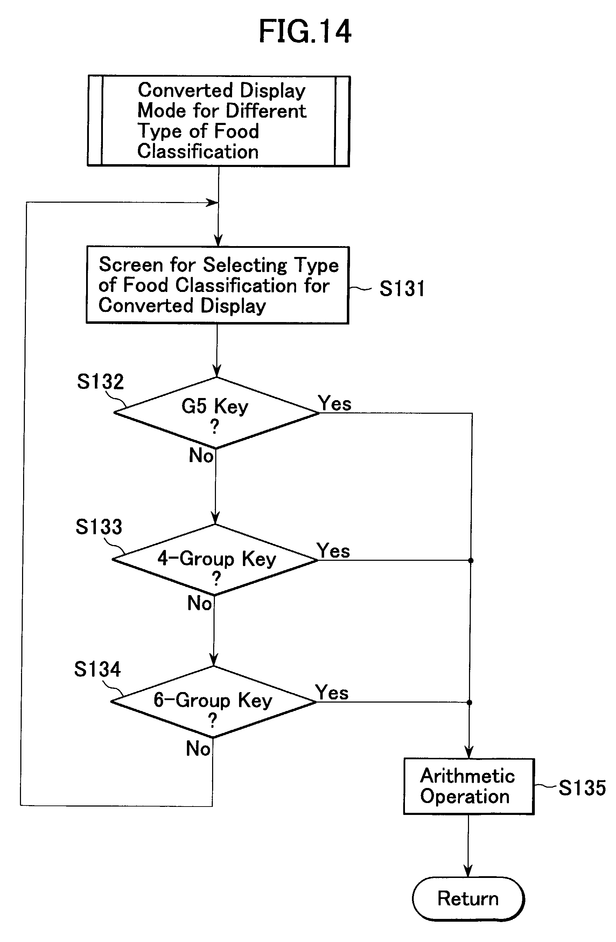

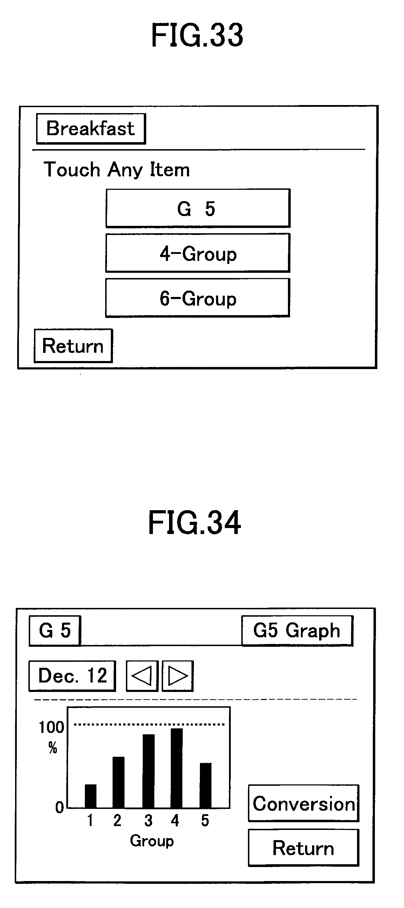

Next, the converted display mode for different type of food classification will be described in more detail. Fig. 14 is a flow chart illustrating the converted display mode for different type of food classification. A display screen for selecting the type of food classification used for the converted display is produced, as shown in Fig. 33 (Step S131). If a "G5" key is depressed (Step S132) then the nutritive substance for the foods ingested is converted into the distribution data in the form of 5-group type of food classification. On the other hand, if a 4-group key is depressed (Step S133), then the nutritive substance for the foods ingested is converted into the distribution data in four groups according to 4-group pointing method. If a 6-group key is depressed (Step S134), then it is converted into the distribution data in six groups.

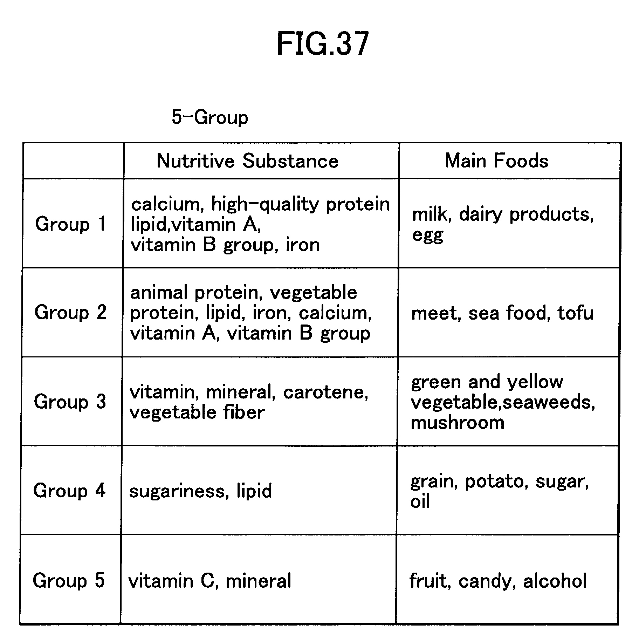

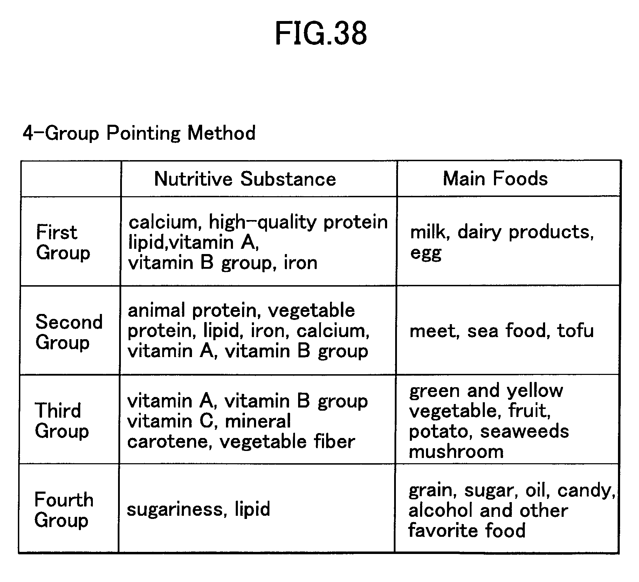

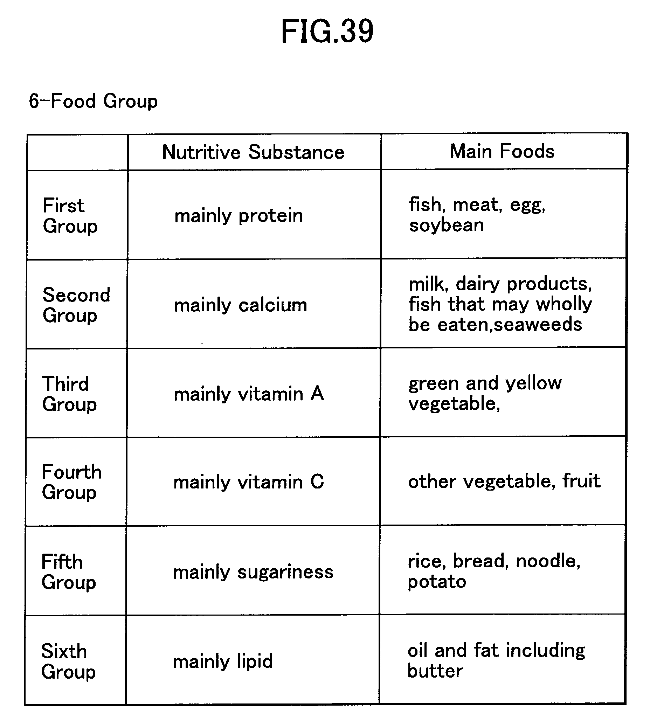

The 5-group type of food classification is a unique grouping method used for the calorie management apparatus according to the present invention in which the foods are classified according to the grouping of nutritive substance, as shown in Fig. 37. On the other hand, the 4-group pointing method is a grouping method proposed by Kagawa Nutrition University of Japan in which the foods are classified, as shown in Fig. 38. And the 6-group type of food classification is a grouping method guided by the Ministry of Health and Welfare in which the foods are classified, as shown in Fig. 39.

Such conversion data is derived based on the information about an amount of nutritive substance per unit amount of each food that is stored in the ROM 12. The foods are classified according to which nutritive substance belonging to which group, as shown in Figs. 37 to 39, to produce the converted distribution data for display.

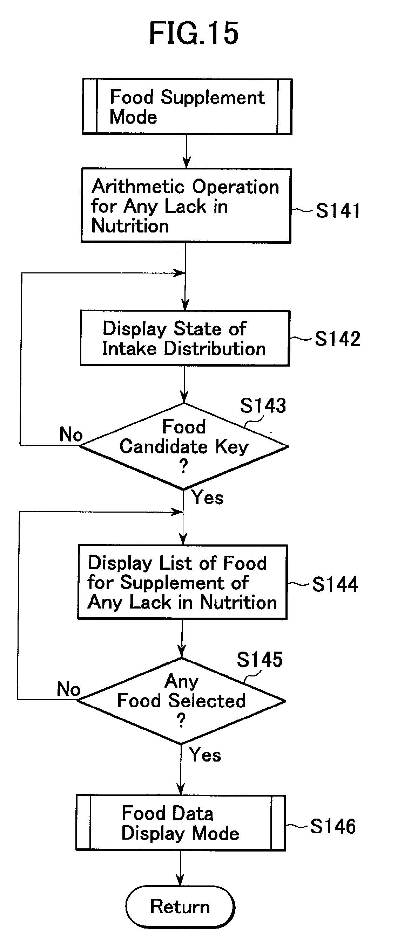

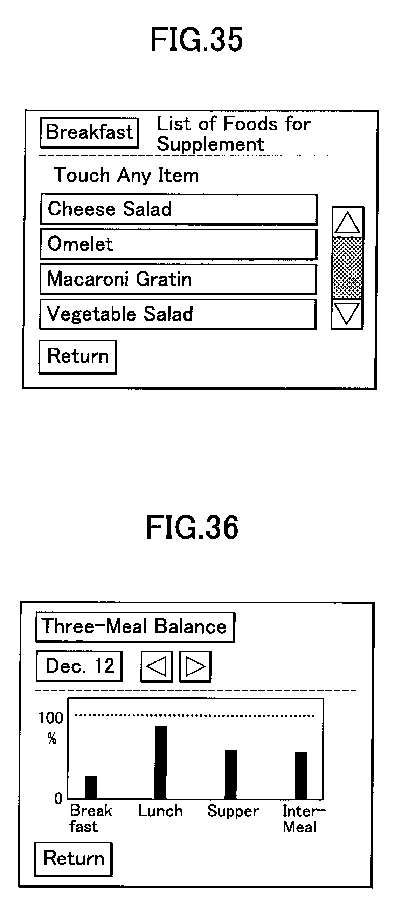

Next, the food supplement mode will be described in more detail. Fig. 15 is a flow chart illustrating the food supplement mode. In such food supplement mode an arithmetic operation is performed for calculating any lack in nutrition for that day from the necessary nutrition for a day (Step S141). Then the state of intake distribution is graphically displayed, as shown in Fig. 34 (Step S142). If a food candidate key is depressed (Step S143) then a list of foods preferable for supplement of lack in nutrition is displayed, as shown in Fig. 35 (Step S144). The list of foods is prepared based on the remainder calorie intake for that day and the food inadequate for the user. In other words, certain foods of which calorie in amount for one person is less than the calorie that is currently allowed for intake are searched for among those stored in the ROM 12 and some of them that have much nutritive substance for supplement of most inadequate food is selected for display. If the calorie intake for that day is already exceeded the necessary calorie then there is no food supplement mode displayed. After selection of the food (Step S145) the routine proceeds to the food data display mode (Step S146).

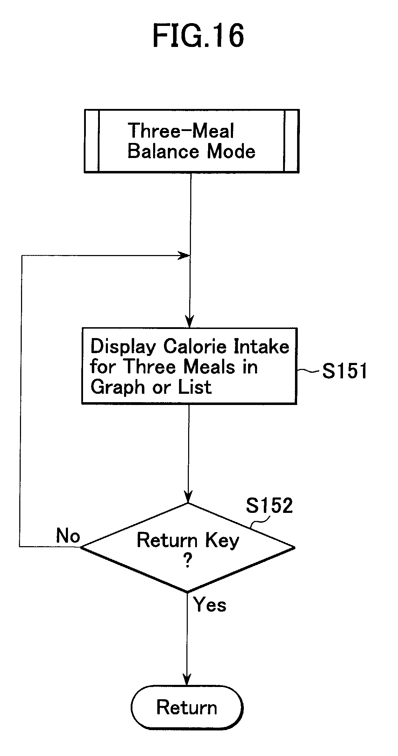

Next, the three-meal balance mode will be described in more detail. Fig. 16 is a flow chart illustrating the three-meal balance mode. In this mode the state of intake distribution among the breakfast, lunch, supper and inter-meal eating is displayed, as shown in Fig. 36 (Step S151). The necessary calorie for a day of the user is allocated to the breakfast, lunch, supper and inter-meal eating at the ratio of 3:3:3.5:0.5, and correspondingly, the actual intake distribution among them is displayed. The display is continued until the return key is depressed (Step S152).

Although the preferred embodiment of present invention has been described above, the present invention may be embodied in any other form without departing from the scope of the claims. For example, if a user has the calorie intake for a day specified by a physician then user may enter it in the initial setting of the apparatus.

In the embodiment as above both of voice input and pen-touch input have been used for entering the food names. Of course, only voice input may be used, but using the pen-touch input as well is more preferable in view of the convenience for the user.

In the embodiment as above the direct input of food names using the pen-touch has been described for manual input means that may be used when the voice recognition is disabled. In addition, there are other conventional methods that may be used in the present invention, such as a character recognized input method in which a pen is used to write a letter on a touch panel which is then recognized; an input method for selecting a food name by including a cross key; and an input method as disclosed in the Japanese Patent Laid-open No. 11-211549.

Furthermore, in the selection of foods for supplement, some nutritive substance for assisting absorption and some foods including such nutritive substance may be displayed, based on the data of foods that have been ingested in the past. For example, it has been said that the iron, if taken with the vitamin C, makes absorption into the body more efficiently. Accordingly if the food including much iron has been taken in the previous meal then the configuration in which the foods including much vitamin C are listed as the food candidate for supplement is very preferable for a user to efficiently absorb the nutritive substance.

It is apparent from the foregoing that a calorie management apparatus of the present invention makes possible to enter the food ingested by using the associated category name, in addition to the name of that food, thereby providing a simplified food name entering capability without the need of remembering the formal name of the food that is preliminary set to the calorie management apparatus.

In addition, the calorie management apparatus of the present invention provides a capability of preliminary storing a combination of foods that are frequently ingested, which allows facilitated use of the apparatus because of no need of entering all the foods every time when they are eaten.

According to such capability of the present invention it is also possible to preliminary store the precise amount of each of the foods included in a specially prepared meal for a specified person, which allows easy entering of the data about such meal of the person.

Moreover, the calorie management apparatus of the present invention can handle a basic unit volume of food, in addition to the weight and calorie of the food. Accordingly, even if the precise weight of the food ingested is not known, the basic unit volume of the food that a user can roughly estimate is used to enter the amount of the food, which allows facilitated entering of the food ingested.

Moreover, the calorie management apparatus of the present invention can be carried with a user to everywhere so that he can use the functions of the apparatus as described above for achieving precise calorie management.

Because of facilitated entering of the food ingested, as described above, there is less error produced in entering the data of food ingested, thereby attaining highly precise calorie management.

Moreover, the calorie management apparatus of the present invention makes possible to manage the calorie intake with both conversion rate of "80kcal = 1 point" and "100kcal = 1 point", thereby providing simplified conversion of calorie intake and easy operation of the apparatus for a user.

Furthermore, because of possibility of substantially no error, more precise management of calorie intake can be attained.

The calorie management apparatus of the present invention makes possible to display the calorie intake according to a plurality of food grouping methods based on the nutritive substance and to understand the state of intake of nutrition from the various points of view, thereby providing good eating habit with balanced nutrition for a user.

The calorie management apparatus of the present invention makes possible to select and inform a user of the food preferable to be taken depending on the remainder calorie that may be taken and any nutritive substance inadequate to the user, whereby he can know the food suitable for balancing the nutrition.

The calorie management apparatus of the present invention makes possible for a user to know the total calorie intake and the nutrition balance produced if he would eat the desired food displayed, with the result that he easily determines whether he may eat it or not.

The calorie management apparatus of the present invention makes possible to modify the selection of the food preferable to be eaten depending on the foods that a use has eaten, so that the user may select any food that he has not yet eaten, which prompts the user to eat a new food or meal. Accordingly, the user is always prompted to eat a new food or meal, which is effective in that the user can challenge the management of calorie intake without lost interested in.

Furthermore, the calorie management apparatus of the present invention makes possible to manage the calorie intake for each of three meals (breakfast, lunch and supper) or four meals (inter-meal eating added) every day, instead of total calorie intake during a longer period such as a whole day, a week or a month, as in the case of the prior art apparatus.

This can avoid any unbalanced meal to leads to an effective diet.

In addition, it is possible for a user to eliminate the habit that he takes no breakfast so that the user is prompted to take the meals orderly

## Claims

1. A calorie management apparatus for calculating and managing a calorie intake, composing:
an input unit;
a storage unit;
a display unit; and
a control unit, wherein
said input unit enters the name and the amount of food ingested,
said storage unit stores the name and the calorie per unit amount of each of the foods in corresponding relation, and further stores the category to which each food belongs,
said display unit displays the result of calorie intake calculated and other information about the food of which name is entered,
said control unit controls calculation of calorie intake, based on the calorie per unit amount of the food stored in said storage unit corresponding to the name of food entered by said input unit and the amount of food also entered by said input unit, and
if the name of food entered by the input unit is one that represents the category of the food stored in the storage unit, then said control unit causes a list of the foods belonging to that category to be displayed on the display unit so that a user selects and enters the food ingested from among the list of the foods displayed.

2. A calorie management apparatus according to claim 1 in which said storage unit further stores an upper level category including a plurality of said categories, and
if the name of food entered by the input unit is one that represents the upper level category stored in the storage unit, then said control unit causes a list of the categories belonging to that upper level category to be displayed on the display unit .

3. A calorie management apparatus for calculating and managing a calorie intake, comprising:
an input unit;
a food group name registration unit;
a storage unit;
a control unit; and
a display unit; wherein
said input unit enters the name and the amount of food ingested,
said food group name registration unit enters a new desired set name for a food group including a plurality of foods,
said storage unit stores the name and the calorie per unit amount of each of the foods in corresponding relation, and further stores the set name for the food group including a plurality of foods entered by said food group name registration unit and the corresponding calorie value,
said control unit controls calculation of calorie intake, based on the calorie per unit amount of the food stored in said storage unit corresponding to the name of food entered by said input unit and the amount of food also entered by said input unit, and allows entering of the food ingested with the new set name for the food group by said input unit, and
said display unit displays the result of calorie intake calculated and other information about the food of which name is entered.

4. A calorie management apparatus according to claim 1 or 3 in which said storage unit further stores a basic unit volume as the unit representing the amount of the food, and
said control unit further controls calculation of calorie of the food ingested, based on the basic unit volume of the food if this is entered by the input unit.

5. A calorie management apparatus according to claim 4 in which said display unit displays the basic unit volume, weight and calorie of each food, and
when either one of the basic unit volume, weight or calorie of the food is entered by the input unit then the control unit causes calculation of the other values than that entered if necessary for displaying them on the display unit.

6. A calorie management apparatus for calculating and managing a calorie intake, comprising:
an input unit;
a storage unit;
a control unit; and
a display unit, wherein
said input unit enters the name and the amount of food ingested,
said storage unit stores the name and the calorie per unit amount of each of the foods in corresponding relation,
said control unit controls calculation of calorie intake, based on the calorie per unit amount of the food stored in said storage unit corresponding to the name of food entered by said input unit and the amount of food also entered by said input unit,
while the name and the amount of the food stored in said storage unit is displayed, said control unit causes confirmation by calculating the calorie intake that would be resulted if such food is eaten, and
said display unit displays the result of calorie intake calculated and other information about the food of which name is entered.

7. A calorie management apparatus according to claim 6 in which it further includes a graphic display unit that graphically illustrates the state of calorie intake for each of food groups after taking a meal.

8. A calorie management apparatus for calculating and managing a calorie intake, comprising:
an input unit;
a storage unit;
a control unit; and
a display unit, wherein
said input unit enters the name and the amount of food ingested,
said storage unit stores the name and the calorie per unit amount of each of the foods in corresponding relation, and further stores the information about contents of nutritive substance classified by food grouping for each of the foods,
said display unit displays the result of calorie intake calculated and other information about the food of which name is entered, and
said control unit controls calculation of calorie intake, based on the calorie per unit amount of the food stored in said storage unit corresponding to the name of food entered by said input unit and the amount of food also entered by said input unit, for calorie intake management according to a plurality of food groups.

9. A calorie management apparatus according to claim 8 in which it further includes a graphic display unit that graphically illustrates the state of calorie intake for each of food groups.
